# EUROPEAN PATENT APPLICATION

(11) **EP 4 483 886 A1**
(43) Date of publication of application: **01.01.2025**
(21) Application number: 23182818.7
(22) Date of filing: 30.06.2023
(51) Int. Cl.: A61K 31/7105, A61K 31/7115, A61P 35/00, A61P 35/04

(54) **RNA COMPOUNDS FOR TREATING PROLIFERATIVE DISORDERS**

(71) Applicant: Johann-Wolfgang-Goethe-Universität Frankfurt am Main, 60323 Frankfurt am Main (DE)
(72) Inventor: STREBHARDT, Klaus, 61462 Königstein i. Ts. (DE); RAAB, Monika, 63549 Ronneburg (DE); KOSTOVA, Izabela, 42115 Wuppertal (DE); BECKER, Sven, 60318 Frankfurt (DE)
(74) Representative: Kuttenkeuler, David

(57) **Abstract**

The invention is based on in vitro transcribed or in vitro synthesized RNA compounds for a translation of tumor suppressor proteins in subjects suffering from a proliferative disease such as cancer. The invention provides the RNA compounds, as well as compositions for their delivery or administration to subjects

## Description

### FIELD OF THE INVENTION

The invention is based on in vitro transcribed or in vitro synthesized RNA compounds for a translation of tumor suppressor proteins in subjects suffering from a proliferative disease such as cancer. The invention provides the RNA compounds, as well as compositions for their delivery or administration to subjects.

### DESCRIPTION

High-grade serous ovarian cancer (HGSOC) is the most lethal gynecologic malignancy and the fifth most common cause of cancer-related death in women. The estimated annual incidence of this disease worldwide is over 200,000 individuals, with approximately 125,000 deaths. These cancers grow very fast, metastasize extensively, and show an aggressive course of the disease. Ovarian cancer (OC) cells stay within the peritoneal cavity and only disseminate to the mesothelium-lined surface1. The current standard of care encompasses cytoreductive surgery, chemotherapy (platinum compounds, paclitaxel, and cyclophosphamide), bevacizumab (Avastin), and PARP (poly-ADP-ribose polymerase) inhibitors. Still, around 70% of patients will suffer from a relapse within three years following surgery and platinum-based chemotherapy and succumb to disease progression.

The TP53 gene plays a key role in human cancer and is the most frequently mutated human tumor suppressor gene mostly through point mutations whereby amino acid substitutions lead to disruption of tumor protein p53 binding to DNA. p53 shields the human genome by counteracting cellular stress and DNA damage. Hence, cells with mutant TP53 can develop an unstable genome, evade apoptosis, and may finally develop into carcinogenesis. Multiple reports showed that tumor cells harboring functionally inactive p53 are resistant to chemotherapeutics since the mechanism of their efficacy is DNA damage leading to the activation of wild-type (wt) p53. The Cancer Genome Atlas Research Network revealed that HGSOC is characterized by TP53 mutations in up to 96% of all cases, leading to an inactive or truncated p53 protein10. Based on the TP53 database (http://www-p53.iarc.fr/) of the International Agency for Research on Cancer (IARC), most TP53 mutations in OC are missense mutations (>87.56%), which mainly cluster at hotspots (codons 175, 248, 273) within the DNA binding domain of p53. While a low prevalence (2-6%) of recurrently mutated genes such as BRCA1, BRCA2, RB1, and NF and a long list of infrequently mutated genes were found in this malignancy, TP53 is, in fact, the only gene that is frequently mutated at the somatic level in HGSOC10. Mutations in TP53 seem to be an early event in tumorigenesis, likely in precursor lesions of OC, supporting the importance of mutated TP53 as a driver of this malignancy.

Recently, RNA-based vaccines have emerged as valuable tools for immunotherapy. Novel strategies based on mRNA therapeutics came into view as efficacious and safe approaches to fighting infectious diseases and cancer based on their advantages such as relatively simple large-scale production, low side effects, and high efficacy. Unlike immune checkpoint blockade setting free immunosuppression and CAR-T cells eliminating cancer cells directly, recently developed cancer vaccines bring about the anticancer immune response via antigen-presenting cells, especially dendritic cells. Endeavors for replacing missing or faulty proteins by mRNA therapeutics have become an attractive strategy in different clinical areas like lung diseases and muscle atrophy, but approaches in cancer are still rare. Whether or not a p53 rescue has therapeutic value highly depends on the tumour indication and/or patient.

Thus, it is an object of the invention to provide novel treatment strategies to tackles proliferative disorders, in particular ovarian cancer.

### BRIEF DESCRIPTION OF THE INVENTION

Generally, and by way of brief description, the main aspects of the present invention can be described as follows:
In a **first aspect,** the invention pertains to a ribonucleic acid (RNA) compound for use in the treatment of a proliferative disease in a subject, wherein the RNA compound encodes an amino acid sequence of a human p53 protein.
In a **second aspect,** the invention pertains a pharmaceutical composition comprising the RNA compound which encodes an amino acid sequence of a human p53 protein.
In a **third aspect,** the invention pertains to a method of treating a proliferative disorder in a subject, the method comprising a step of administering to the subject an RNA compound which encodes an amino acid sequence of a human p53 protein.
In a **fourth aspect,** the invention pertains a method of manufacturing a medicament for use in the treatment of proliferative disorder in a subject, wherein the method comprises a step of formulating an RNA compound which encodes an amino acid sequence of a human p53 protein as a medicament.

### DETAILED DESCRIPTION OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

In **the first aspect,** the invention pertains to a ribonucleic acid (RNA) compound for use in the treatment of a proliferative disease in a subject, wherein the RNA compound encodes an amino acid sequence of a human p53 protein, or a variant thereof.

Cellular tumor antigen p53 is encoded by the TP53 gene and its sequence and coding gene sequences can be derived via the UniProt database in the version of June 20 2023, under the accession No. P04637. The amino acid sequence of wild type p53 is also included herein as SEQ ID NO: 1.

A preferred embodiment of the invention pertains therefore to an RNA compound which comprises, or consists essentially of a p53 mRNA

The RNA compound according to the invention has a sequence having at least 80% sequence identity to a nucleic acid sequence encoding a human p53 protein (SEQ ID NO: 1). In the context of the invention, the term "sequences having at least 80% sequence identity" comprises sequences having at least 85 %, at least 90 %, at least 95 %, at least 96 %, at least 97 %, at least 98 %, at least 99 %, at least 99.5 %, most preferably 100 % sequence identity to the respective sequence. In all cases, the identity is the identity over the total length of the sequences.

Preferably, an RNA compound of the invention comprises an RNA sequence of SEQ ID NO: 3, or a sequence that is at least 80%, more preferably 85%, 90%, 95%, 96%, 97%, 98%, 99% identical to the sequence of SEQ ID NO: 3.

In one aspect, the RNA compound according to the invention is a variant of the RNA compound having a sequence encoding a protein according to SEQ ID NO: 1, i.e. human p53. The term "variant" herein refers to biologically active derivatives of the respective RNA. In general, the term "variant" refers to molecules having a native sequence and structure with one or more additions, substitutions (generally conservative in nature) and/or deletions, relative to the native molecule, so long as the modifications do not destroy biological activity, and which are "substantially homologous" to the reference molecule. In general, the sequences of such variants will have a high degree of sequence homology to the reference sequence, e.g., sequence homology of more than 50%, generally more than 60%-70%, even more particularly 80%-85% or more, such as at least 90%-95% or more, when the two sequences are aligned. In case of p53, a variant thereof shows a similar capability as a tumor suppressor protein.

In a further embodiment, the invention relates to an RNA compound obtainable by transcription of a DNA sequence having at least 80% sequence identity to nucleic acid sequence encoding for a amino acid sequence of SEQ ID NO: 1, or variant thereof, for use as a medicament. In a preferred embodiment, the RNA compound is for the use in the treatment of cancer.

The term "obtainable by transcription of a DNA sequence" is herein understood as meaning that the RNA compound is the transcription product or transcript of the respective DNA. Thus, the term "RNA compound obtainable by transcription of a DNA sequence having at least 80% sequence identity to a sequence encoding amino acid sequence of SEQ ID NO: 1 refers to any RNA compound that can be transcribed from a DNA sequence having a sequence that encodes a protein with an amino acid sequence that is at least 80% identical to the sequence identity to SEQ ID NO: 1, or any functional variant thereof. Comprised are any RNA compounds, wherein the RNA compound according to the invention may have been processed by undergoing posttranscriptional modifications such as RNA methylation, folding, cleavage or wrapping into extracellular cargo vesicles such as exosomes.

The term "obtainable by transcription of a DNA sequence" is herein understood not to be limited with regard to the source of the RNA molecule. The RNA molecules according to the invention, including the RNA molecules used in the pharmaceutical compositions according to the invention, can be in vivo transcribed RNA molecules purified from cells or in vitro transcribed RNA.

The skilled person knows how RNA can be purified from cells or transcribed in vitro. For example, RNA purification can be performed by using TRIzol Reagent (Invitrogen, CA, catalogue no. 15596018). For this, samples are lysed and homogenized in the appropriate volume of TRIzol Reagent. Samples are incubated at room temperature for 5 minutes, then 1/5 of the volume of chloroform is added, incubated and mixed vigorously. To separate the phases samples are centrifuged for 15 minutes at 13 000 x g at 18°C. The aqueous phase containing the RNA is transferred to a new tube and RNA is precipitated using isopropanol. The resulting pellet is washed using ice cold 70% ethanol, air dried and dissolved in a suitable buffer.

The RNA compounds according to the invention may be purified from any eukaryotic cell expressing p53 artificially or naturally, or may be purified from a transcription in a cell free system. Likewise, RNA purification can be performed using commercially available kits such as RNeasy Mini Kit (Qiagen, Germany, catalogue no. 74106) or Dynabeads^{™} mRNA DIRECT^{™} Purification Kit (Invitrogen, CA, catalogue no. 61012).

The RNA compound for use of the invention is preferred, wherein the RNA compound encoding the amino acid sequence comprises the nucleotide sequence of SEQ ID NO: 2 (RNA sequence preferred), or a nucleotide sequence having at least 99%, 98%, 97%, 96%, 95%, 90%, 85%, or 80% identity to the nucleotide sequence of SEQ ID NO: 2.

The RNA compound of the invention is preferably an in vitro transcribed (IVT) RNA or is a synthetically polymerized RNA.

The RNA compound of the invention may comprise the classical sequence motifs of messenger RNAs. However. The RNA molecules of the invention may also include various additional modifications or mutations in order to increase translation, reduce immunological adverse effects, or increase RNA stability in general. Many such modifications are well known in the art and are not individually listed herein for sake of conciseness.

For example, an RNA compound of the invention may comprise a poly-A sequence in operable linkage to the sequence encoding the amino acid sequence. Preferably, when used, a poly-A sequence comprises at least 100 nucleotides. A poly-A sequence is usually located 3' of the sequence encoding the amino acid sequence.

By a further embodiment, the at least one RNA of the composition disclosed herein preferably comprises at least one of the following structural elements: a 5'- and/or 3'- untranslated region element (UTR element), particularly a 5'-UTR element which comprises or consists of a nucleic acid sequence which is derived from the 5'-UTR of a p53 gene or from a fragment, homolog or a variant thereof, or a 5'- and/or 3'-UTR element which may be derivable from a gene that provides a stable mRNA or from a homolog, fragment or variant thereof; a histone stem-loop structure, preferably a histone stem-loop in its 3' untranslated region; a 5'-CAP structure; a poly-A tail (poly(A) sequence); or a poly(C) sequence.

In another embodiment the RNA compound comprises a 5' cap structure. 5 cap structures are well known in the art, but preferred Caps are selected from a Cap-0. Cap-1 or Cap-2 structure, preferably which is m7G(5')ppp(5') (2'OMeA)pG.

Also preferred is an embodiment, wherein the RNA compound comprises a 5' UTR comprising an eIF4G aptamer.

Preferred RNA compounds of the invention comprise at least one nucleotide which is a modified nucleotide, such as pseudo uridine or 5-methylcytidine, for example wherein the modified nucleotide is a replacement of at least one uracil with pseudo uridine, preferably is a replacement of all uracil positions with pseudo uridine.

An RNA compound in accordance with the invention in preferred embodiments is derived from a mammalian vector based expression of p53 mRNA and induces apoptosis in cancer cells in accordance with the invention.

The RNA compound for use of any one of claims 1 to 10, wherein the RNA compound is administered to the subject by administering a pharmaceutically acceptable liposomal formulation comprising the RNA-compound.

A proliferative disease in context of the invention is a tumor disease. A tumor disease is in particular an ovarian cancer, and most preferred is a High-grade serous ovarian cancer (HGSOC). A tumor of the invention may be a metastatic tumor and/or a recurrent (relapsed) tumor.

In a preferred embodiment of the invention a treatment in accordance with the first aspect involves the administration of the RNA compound to the subject, p53 translation via the RNA compound in tumor tissue or tissue adjacent to a tumor, and thereby induction of apoptosis in tumor cells specifically.

A subject to be treated in accordance with the invention in some embodiments received a surgery.

Certain embodiments specifically pertain to the disease HGSOC and wherein the subject received or receives (while administrating the RNA compound of the invention, e.g by rinsing) a tumor debulking therapy in the intra peritoneal cavity. Thus, in certain embodiments the RNA compound is administered to the subject intraperitoneal.

In some embodiment, the subject received or receives at least one additional anticancer therapy. For example, the additional anticancer therapy is chemotherapy, such as a chemotherapy involving administration of one or more platin compound. A chemotherapy administered to a subject in context of the invention includes administration of paclitaxel in combination with cisplatin or cisplatin in combination with cyclophosphamide.

A preferred embodiment of the invention pertains to the RNA compound for use in treatment as disclosed, wherein the proliferative disease is HGSOC, the subject received or receives debulking therapy, and wherein the RNA compound is administered into the intraperitoneal cavity of the subject.

Further provided in another aspect is a pharmaceutical composition, the composition comprising the RNA compound recited above, and at least one pharmaceutically acceptable excipient.

The invention also relates to a pharmaceutical composition comprising as active ingredient an RNA molecule obtainable by transcription of a DNA sequence having at least 80% sequence identity to SEQ ID NO: 3. In one embodiment, the pharmaceutical composition comprises as active ingredient an RNA molecule having at least 80% sequence identity to a nucleic acid sequence encoding for a protein of SEQ ID NO: 1.

It is herein understood that a pharmaceutical composition comprises a therapeutically effective amount of the active ingredient as well as pharmacological excipients. The person skilled in the art knows how to formulate a pharmaceutical composition comprising an RNA molecule. In particular, the person skilled in the art is aware of methods for stabilizing an RNA molecule in a pharmaceutical composition.

In the pharmaceutical composition according to the invention, the RNA molecule may be encapsulated in lipid nanoparticles, cellular or synthetic exosome mimics or in virus-like particles.

Herein, the term "lipid nanoparticles" is defined as molecules that are spherical in shape and comprise a solid lipid core stabilized by a surfactant. The core lipids can be steroids, fatty acids, acylglycerols, waxes, and combinations of them. Surfactants may be biological membrane lipids such as phospholipids, sphingomyelins and bile salts (e.g., sodium taurocholate). All of these may be utilized as stabilizers in the lipid nanoparticles used for pharmaceutical compositions of the invention.

Herein, the term "cellular or synthetic exosome mimics" is defined as nano-sized vesicles (exosomes), derived or purified from cells with modifications (cellular exosome mimics) or generated by artificial methods like cell extrusion (synthetic exosome mimics) that serve as cargos to deliver proteins, nucleic acids or other cellular components to neighbouring or distant cells.

Herein, the term "virus-like particles" is defined as multiprotein structures that closely resemble the organization and conformation of viruses but contain no viral genetic material.

Also disclosed is a method of treating a proliferative disorder in a subject, the method comprising a step of administering to the subject an RNA compound herein.

Also disclosed is a method of manufacturing a medicament for use in the treatment of proliferative disorder in a subject, wherein the method comprises a step of formulating an RNA compound as recited herein as a medicament. The method comprises a step of in-vitro transcribing an RNA compound as defined herein, in some preferred embodiments.

Further, the method may comprise a step of formulating the RNA compound as a liposomal composition, wherein the liposome encapsulates the RNA compound.

The invention also is described by the following itemized embodiments, which shall be understood and interpreted in context of this complete disclosure, in particular in view of the various definitions and explanations and the examples provided herein.

Item 1:A ribonucleic acid (RNA) compound for use in the treatment of a proliferative disease in a subject, wherein the RNA compound encodes an amino acid sequence of a human p53 protein.

Item 2: The RNA compound for use of item 1, wherein the RNA compound encoding the amino acid sequence comprises the nucleotide sequence of SEQ ID NO: 1 (RNA sequence preferred), or a nucleotide sequence having at least 99%, 98%, 97%, 96%, 95%, 90%, 85%, or 80% identity to the nucleotide sequence of SEQ ID NO: 1.

Item 3: The RNA compound for use of item 1 or 2, wherein the RNA compound is an in vitro transcribed (IVT) RNA or is a synthetically polymerized RNA.

Item 4: The RNA compound for use of any one of items 1 to 3, wherein the RNA compound comprises a poly-A sequence in operable linkage to the sequence encoding the amino acid sequence.

Item 5: The RNA compound for use of item 4, wherein the poly-A sequence comprises at least 100 nucleotides.

Item 6: The RNA compound for use of item 4 or 5, wherein the poly-A sequence is located 3' of the sequence encoding the amino acid sequence.

Item 7: The RNA compound for use of any one of items 1 to 6, wherein the RNA molecule comprises a 5' cap structure, such as a Cap-0. Cap-1 or Cap-2 structure, preferably which is m7G(5')ppp(5') (2'OMeA)pG.

Item 8: The RNA compound for use of any one of items 1 to 7, wherein the RNA compound comprises a 5' untranslated region (UTR),

Item 9:The RNA compound for use of item 8, wherein the 5' UTR comprises an elF4G aptamer.

Item 10: The RNA compound for use of any one of items 1 to 9, wherein at least one nucleotide is a modified nucleotide, such as pseudo uridine or 5-methylcytidine, for example wherein the modified nucleotide is a replacement of at least one uracil with pseudo uridine, preferably is a replacement of all uracil positions with pseudo uridine.

Item 11: The RNA compound for use of any one of items 1 to 10, wherein the RNA compound is administered to the subject by administering a pharmaceutically acceptable liposomal formulation comprising the RNA-compound.

Item 12: The RNA compound for use of any one of items 1 to 11, wherein the proliferative disease is a tumor disease.

Item 13: The RNA compound for use of item 12, wherein the tumor disease is ovarian cancer.

Item 14: The RNA compound for use of item 13, wherein the ovarian cancer is High-grade serous ovarian cancer (HGSOC).

Item 15: The RNA compound for use of any one of items 12 to 14, wherein the tumor is a metastatic tumor.

Item 16: The RNA compound for use of any one of items 12 to 15, wherein the tumor is a relapsed tumor.

Item 17: The RNA compound for use of any one of items 1 to 16, wherein the subject received a surgery.

Item 18: The RNA compound for use of any one of items 1 to 17, wherein the disease is HGSOC and the subject received tumor debulking therapy in the intra peritoneal cavity.

Item 19: The RNA compound for use of any one of items 1 to 18, wherein the RNA compound is administered to the subject intraperitoneal.

Item 20: The RNA compound for use of any one of items 1 to 19, wherein the subject received or receives at least one additional anticancer therapy.

Item 21: The RNA compound for use of item 20, wherein the additional anticancer therapy is chemotherapy.

Item 22: The RNA compound for use of item 21, wherein the chemotherapy involves administration of one or more platin compound.

Item 23: The RNA compound for use of item 21 or 22, wherein the chemotherapy is an administration of paclitaxel in combination with cisplatin or cisplatin in combination with cyclophosphamide.

Item 24: The RNA compound for use of any one of items 1 to 23, wherein the proliferative disease is HGSOC, the subject received debulking therapy, and wherein the RNA compound is administered into the intraperitoneal cavity.

Item 25: A pharmaceutical composition, the composition comprising the RNA compound recited in any one of items 1 to 24, and at least one pharmaceutically acceptable excipient.

Item 26: The pharmaceutical composition of item 25, which is a liposomal formulation of the RNA compound.

Item 27: The pharmaceutical composition of item 25 or 26, for a use in therapy as recited in any one of items 1 to 24.

Item 28: A method of treating a proliferative disorder in a subject, the method comprising a step of administering to the subject an RNA compound recited in any one of items 1 to 24.

Item 29: The method of item 28, wherein treatment is a use as recited in any one of items 1 to 24.

Item 30: A method of manufacturing a medicament for use in the treatment of proliferative disorder in a subject, wherein the method comprises a step of formulating an RNA compound as recited in any one of items 1 to 24.

Item 31: The method of item 30, wherein the method comprises a step of in-vitro transcribe an RNA compound as defined in any one of the preceding items.

Item 32: The method of item 30 or 31, wherein the method comprises a step of formulating the RNA compound as a liposomal composition, wherein the liposome encapsulates the RNA compound.

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of", both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

It is to be understood that application of the teachings of the present invention to a specific problem or environment, and the inclusion of variations of the present invention or additional features thereto (such as further aspects and embodiments), will be within the capabilities of one having ordinary skill in the art in light of the teachings contained herein.

Unless context dictates otherwise, the descriptions and definitions of the features set out above are not limited to any particular aspect or embodiment of the invention and apply equally to all aspects and embodiments which are described.

All references, patents, and publications cited herein are hereby incorporated by reference in their entirety.

### BRIEF DESCRIPTION OF THE FIGURES AND SEQUENCES

The figures show:

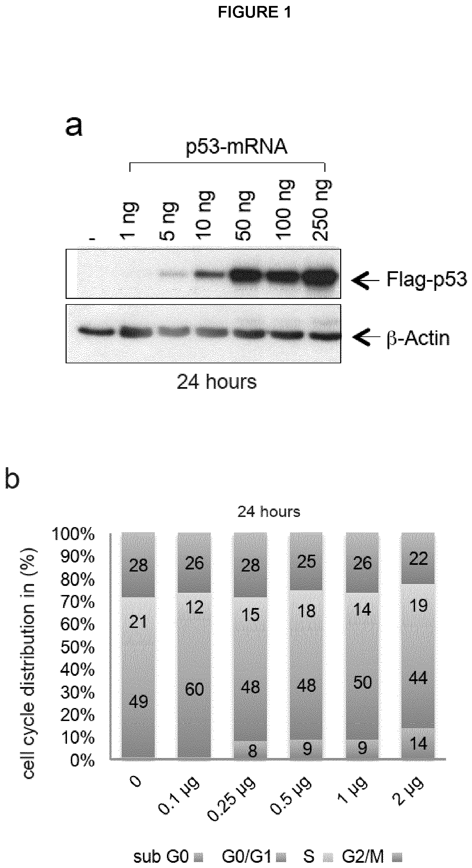

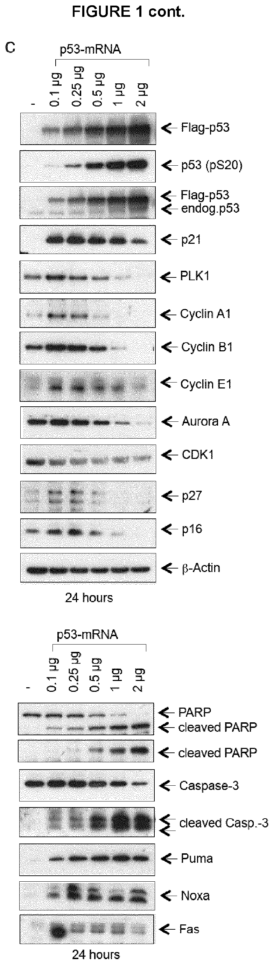

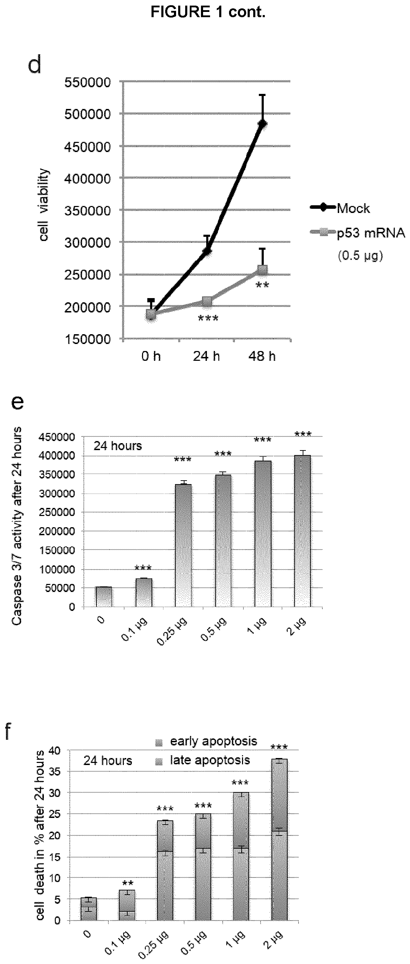

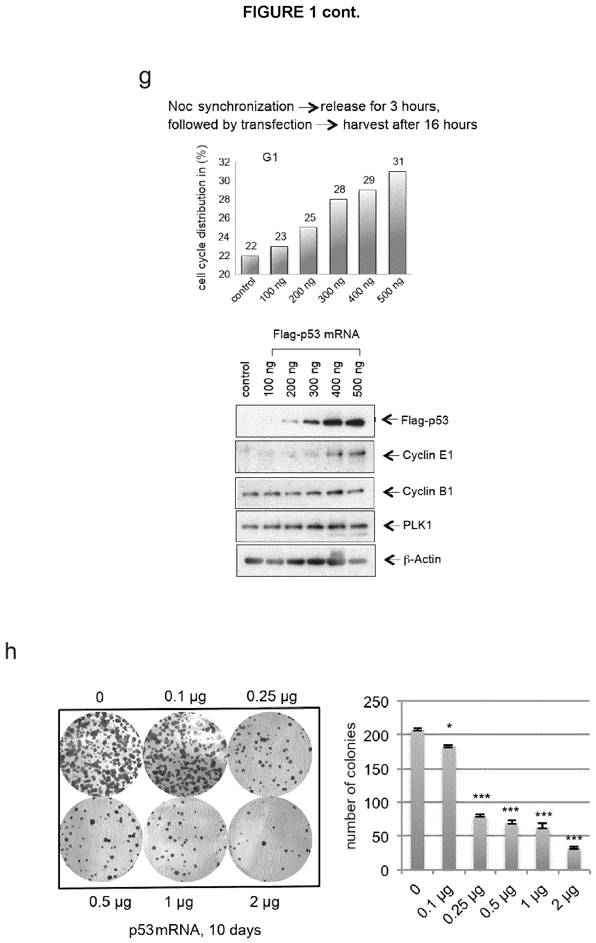

**Figure 1****:** shows liposomal transfection of *in vitro*-transcribed wt p53 mRNA induces cell cycle arrest and apoptosis in the HGSOC cell line OVCAR-8 cells. **a**, OVCAR-8 cells were transfected with increasing amounts of liposomal wt, *in vitro*-transcribed (IVT) Flag-tagged p53 mRNA. Cell lysates were subjected to WB using Flag- and β-Actin-antibodies. **b**, The dose (transfected p53 mRNA)-dependent cell cycle distribution of OVCAR-8 cells 24 h following liposomal transfection is represented as a bar graph. **c,** (upper) Cell lysates were subjected to WB for key mediators of cell cycle regulation using Flag-, p53 (pS20)-, p53-, p21-, PLK1-, Cyclin A1-, Cyclin B1-, Cyclin E1-, Aurora A-, CDK1-, p27-, p16- and β-Actin-antibodies. (lower) The same cell lysates were subjected to WB for regulators of apoptosis using PARP-, Caspase-3-, Puma-, Noxa-, and Fas-antibodies. **d,** Cells were mock- or 0.5 µg p53 mRNA-transfected, and proliferation was measured using a CellTiter-Blue Cell Viability assay, 24 h post-treatment. ***P* < 0.01, ****P* < 0.001, Student's t-test, unpaired and two-tailed, **e,** The dose-dependent Caspase-3/7 activities were determined using a Caspase-Glo 3/7 assay. ****P* < 0.001, Student's t-test, unpaired and two-tailed. **f,** Graphical representation of Annexin V positive (%) cells. FACS analysis of transfected cells, gated for Annexin V positive (early apoptotic), and Annexin V/7AAD positive (late apoptosis) populations is demonstrated. [*n* = 3 for each dose; *** = <0.001 ]. **g,** (upper) The dose-dependent cell cycle distribution of Nocodazole-synchronized cells is represented as a bar graph. (*n* = 3) (lower) Cell lysates were subjected to WB using Flag-, Cyclin E1-, Cyclin B1-, PLK1-, and β-Actin-antibodies. **h,** (left) Representative images show the number of 3D colonies (dose-dependent) on day 10. (right) The dose-dependent distribution of colonies is represented as bar graph. (*n* = 3) **P* < 0.05, ****P* < 0.001, Student's t-test, unpaired and two-tailed.

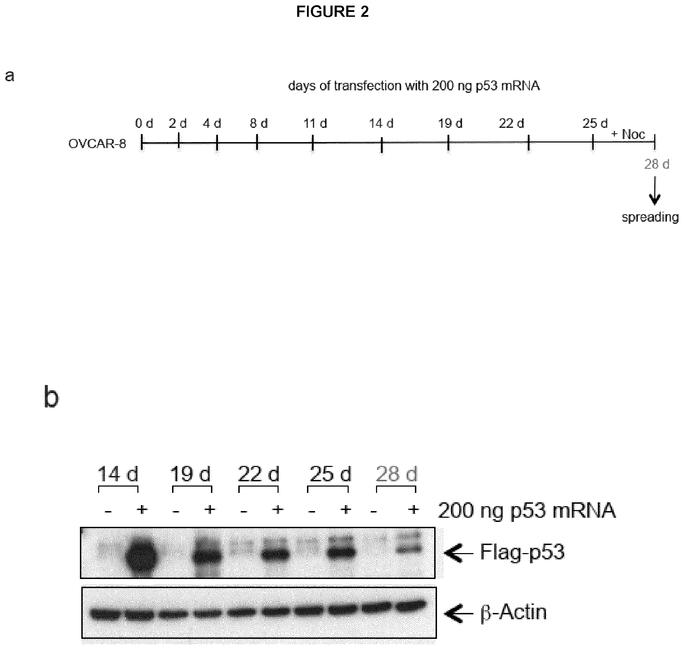

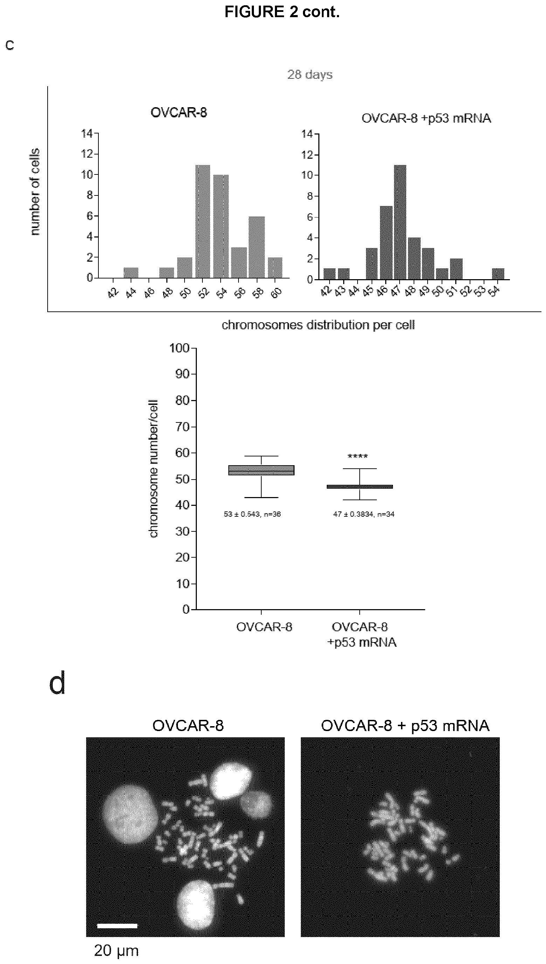

**Figure 2****:** shows restoring p53 function reduces numerical chromosomal instability (nCIN) in OVCAR-8 cells. **a,** Treatment schedule. OVCAR-8 cells were kept in culture for 28 d and treated with 200 ng p53 mRNA every 48 h. Metaphase spreadings of untreated controls and treated OVCAR-8 cells were prepared at the end of the incubation. Chromosomes were stained with Hoechst. **b,** Western blot using Flag- and β-Actin-antibodies. **c,** (upper) Histogram plots of the distribution of chromosome numbers in controls and cells treated with 200 ng p53 mRNA. (lower) Quantification of the number of chromosomes in cells with/without p53 mRNA-treatment. (mean ± SD). The results were analyzed statistically. **** *p* < 0.001. **d,** Representative images of metaphase chromosome spreads. Scale bar = 20 µm.

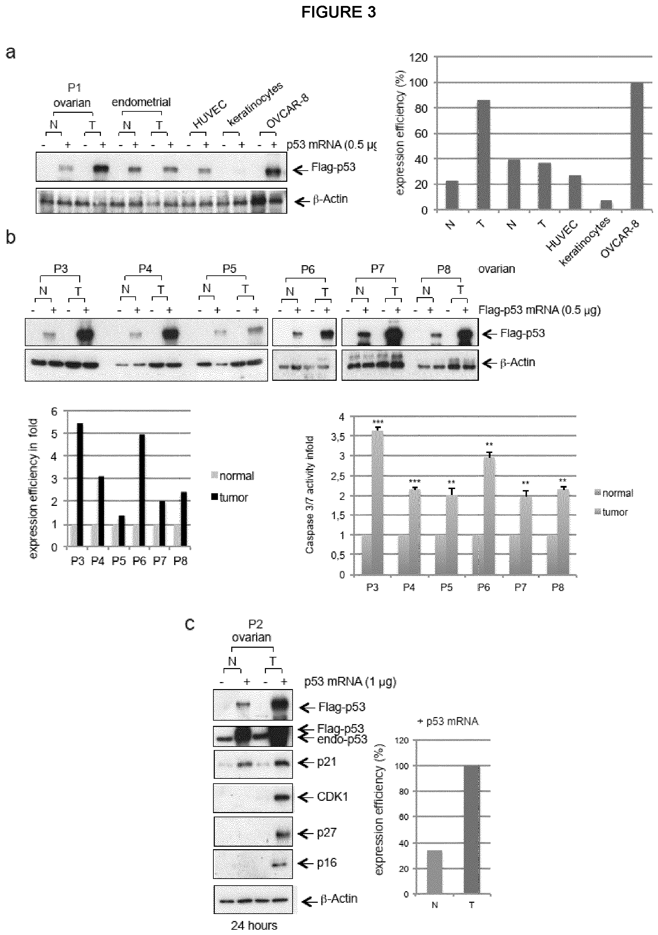

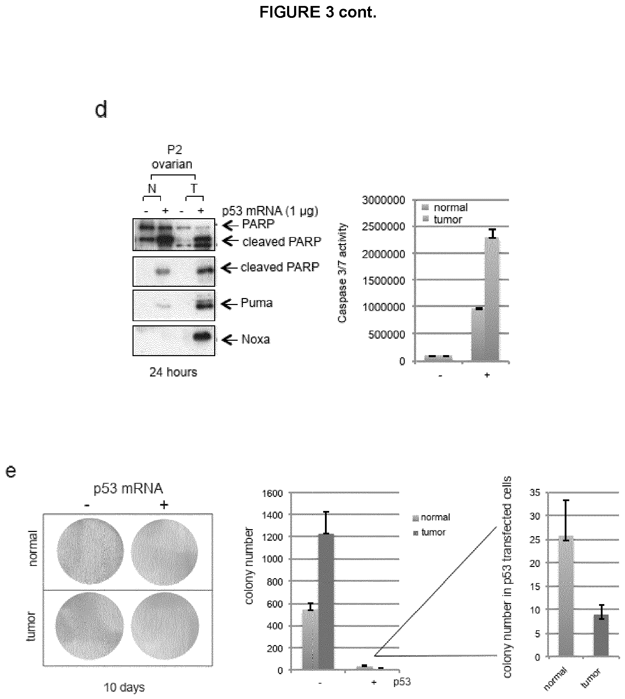

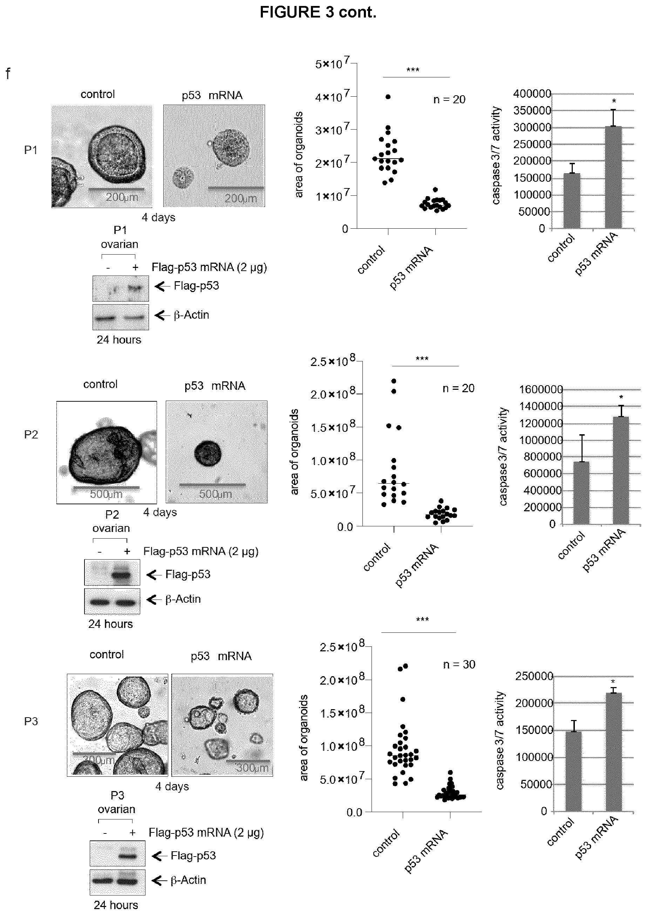

**Figure 3****:** shows liposomal transfection of p53 mRNA results in higher p53 expression in primary HGSOC cells compared to their normal counterparts, **a,** Primary human cells of different origins (tumor, normal) and OVCAR-8 cells were transfected with p53 mRNA. (left) Cell lysates were subjected to WB using Flag- and β-Actin-antibodies. (right) The normalized levels of p53 expression are represented as bar graphs. **b,** (upper) Primary human HGSOC cells (tumor, normal) derived from patients P3-8 were transfected with 0.5 µg p53 mRNA. Cell lysates were subjected to WB using Flag- and β-Actin-antibodies. (lower left) Expression efficacy was depicted as bar graphs. (lower right) Caspase-3/7 activities were determined using a Caspase-Glo 3/7 assay. ***P* < 0.01, ****P* < 0.001, Student's t-test, unpaired and two-tailed. **c,** (left) Lysates of mock- or p53 mRNA-transfected normal ovarian or tumor HGSOC (patient 2) cells were subjected to WB using Flag-, p53-, p21-, CDK1-, p27-, p16-, and β-Actin-antibodies. (right) The normalized levels of p53 expression are represented as a bar graph. **d,** (left) The same cell lysates were subjected to WB using PARP-, Puma-, and Noxa-antibodies. (right) Caspase-3/7 activities of mock- or p53 mRNA-transfected normal and tumor HGSOC cells were determined using a Caspase-Glo 3/7 assay. (n=3) **e,** (left) Representative images show the 3D colonies of transfected cells on day 10. (right) The number of colonies is represented as bar graphs. (*n* = 3) **f,** (left) Representative images show organoids on day 4. (middle) Size of organoids was determined using the imaged Software (n=8) ****P* < 0.001, Student's t-test, unpaired and two-tailed. (right) Caspase-3/7 activities of HGSOC organoids mock- or 2 µg p53 mRNA-transfected were determined using a Caspase-Glo 3/7 assay. (n=3), **P* < 0.05, Student's t-test, unpaired and two-tailed. (lower) Lysates of HGSOC organoids (patient 1-3) mock- or p53 mRNA-transfected were subjected to WB using Flag-, and β-Actin-antibodies.

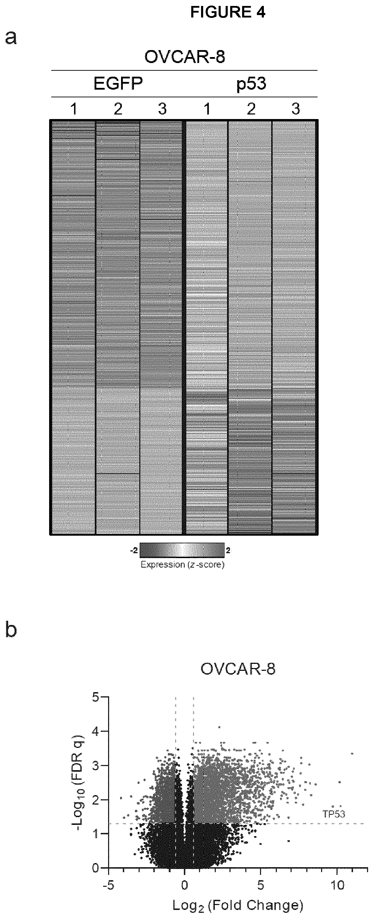

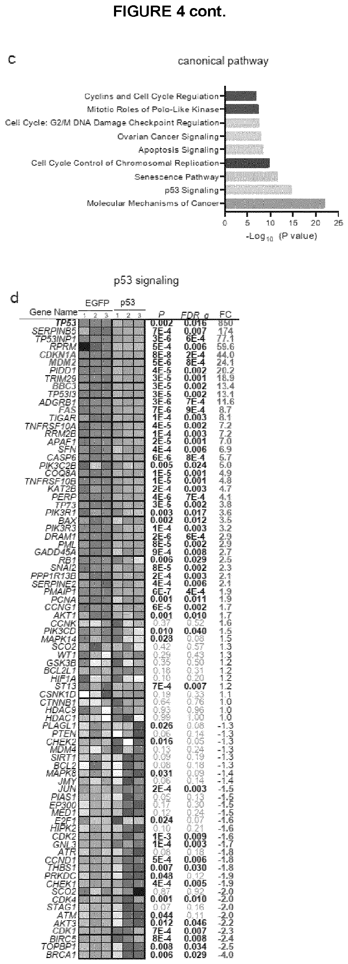

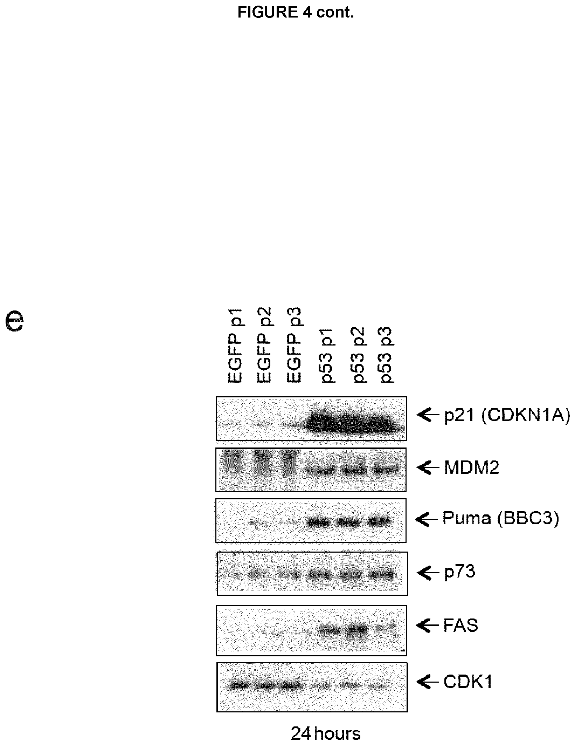

**Figure 4****:** shows identification of differentially expressed genes in p53 mRNA-transfected OVCAR-8 cells compared to mock-transfected counterparts, **a,** Heatmap of normalized gene expression represented by *z* scores of OVCAR-8 cells treated with mock (EGFP mRNA) or p53 mRNA from three independent RNA-seq experiments (Student's *t* tests or Welch tests, depending on the variance, corrected by false-discovery rate [FDR] *q* adjusted *P*-value <0.05 and absolute fold change [FC] >1.5). **b,** Volcano plot showing the differentially-expressed genes (DEGs) in the RNA-seq between OVCAR-8 cells treated with EGFP *vs*. p53 mRNA (red dots=upregulated, blue dots=down-regulated). **c,** Selected overrepresented Ingenuity pathways from pathway enrichment analysis of DEGs with FOR-adjusted *P*-values <0.05 and FC >1.5. Pathways with a positive *z* score are depicted in orange; pathways with a negative z score are depicted in blue; and pathways with no specific pattern are depicted in gray. **d,** Heatmap of all genes corresponding to the "p53 signaling pathway" from Ingenuity Pathway Analysis (IPA), indicating the *P* value, FOR-adjusted *P* value and fold change (FC) for the comparison of EGFP with p53 mRNA. Genes with validation by WB are highlighted in red. **e,** Cell lysates were subjected to WB for p53 signaling pathways using p21, MDM2, Puma, p73, Fas, CDK1, and β-Actin antibodies.

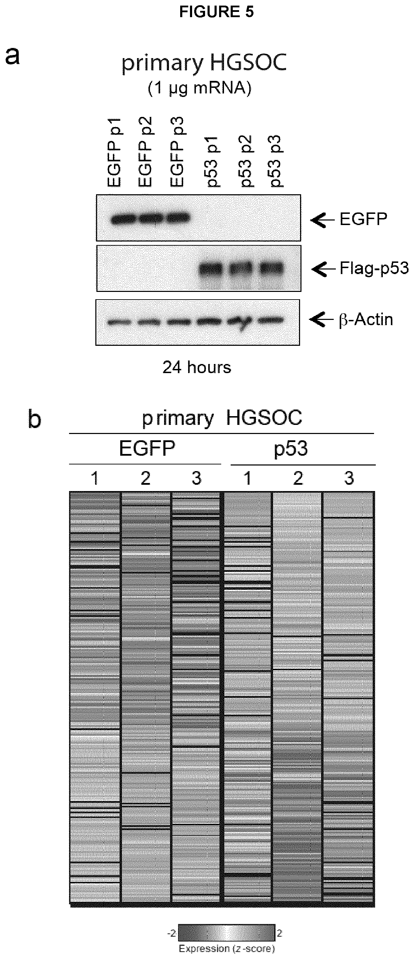

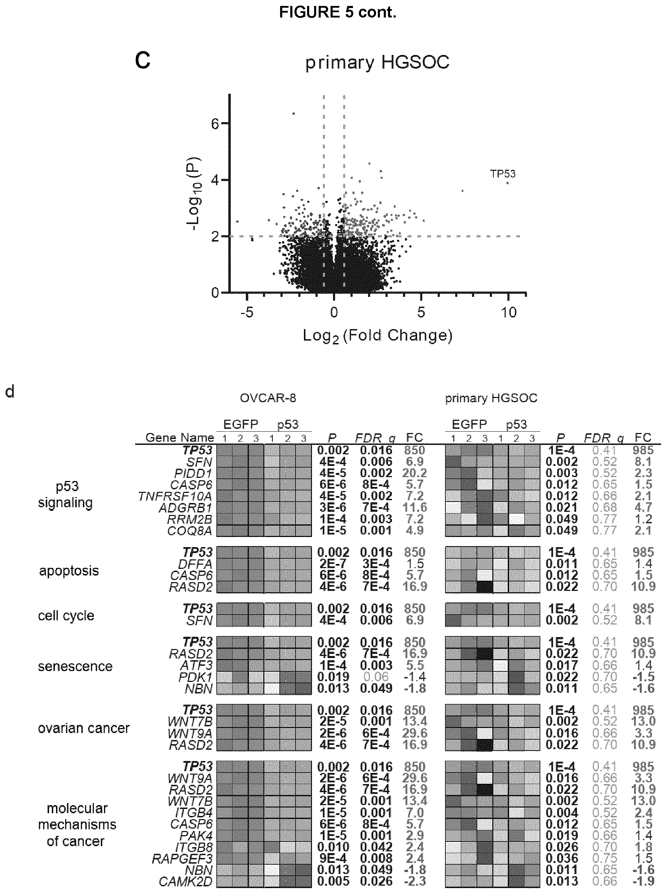

**Figure 5****:** shows Analysis of differentially expressed genes in primary HGSOC cells. **a,** Primary HGSOC cells from patient 1 were transfected with p53 mRNA or EGFP-mRNA. Cell lysates were subjected to WB using EGFP, Flag, and β-Actin antibodies. **b-c,** Heatmap (**b**) and Volcano plot (**c**) showing the differentially-expressed genes (DEGs) in the RNA-seq between primary HGSOC cells treated with EGFP vs. p53 mRNA (*P* value <0.01 and absolute fold change (FC) >1.5) (red dots=up-regulated, blue dots=down-regulated). **d,** Transcriptome analysis of DEGs in common with OVCAR-8 and primary HGSOC cells.

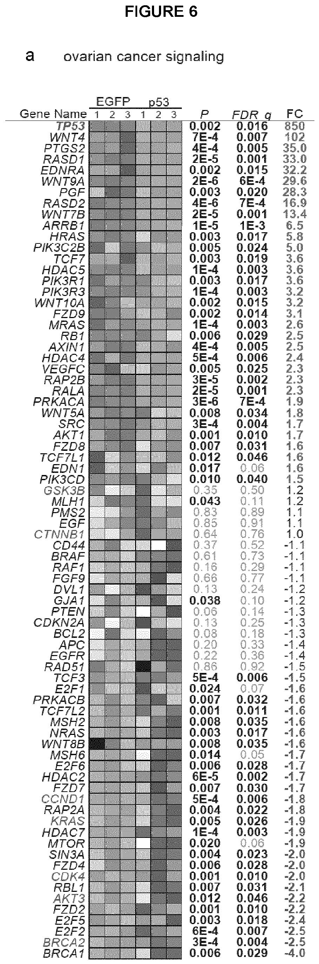

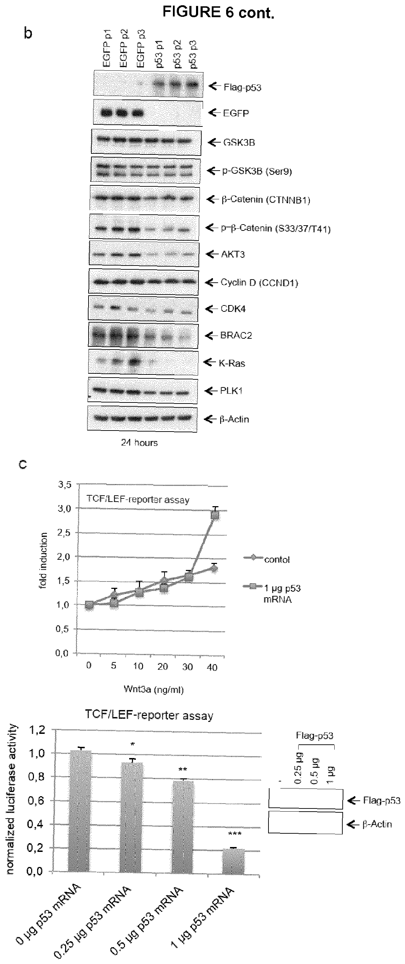

**Figure 6****:** shows Ovarian cancer signaling pathway upregulation upon treatment of OVCAR-8 cells with IVT p53 mRNA. **a,** Heatmap of all genes corresponding to the OC Signaling pathway from Ingenuity Pathway Analysis (IPA), indicating the *P* value, FOR-adjusted *P* value and fold change (FC) for the comparison of EGFP with p53 mRNA in OVCAR-8 cells. Ingenuity Pathway Analysis of significant genes with FOR-adjusted *P*-values <0.05 and FC >1.5 between EGFP and p53 mRNA treatments in OVCAR-8 cells. Downregulated genes are displayed in blue/green (predicted/measured) and upregulated genes in orange/red (predicted/measured). **b,** WB analysis using Flag-, EGFP-, GSK3B-, p-GSK3B-, β-Catenin-, p-β-Catenin-, AKT3-, Cyclin D-, CDK4-, BRCA2-, K-Ras-, PLK1-, and β-Actin-antibodies. **c,** (upper) TCF/LEF luciferase reporter responses to Wnt-3a stimulation of cells. (lower) TCF/LEF luciferase reporter responses of cells transfected with increasing doses of p53 mRNA.

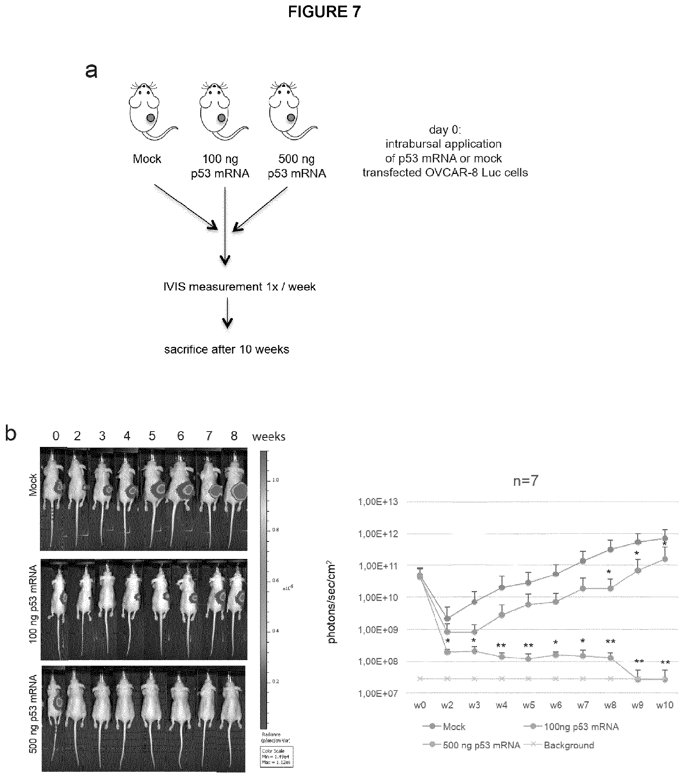

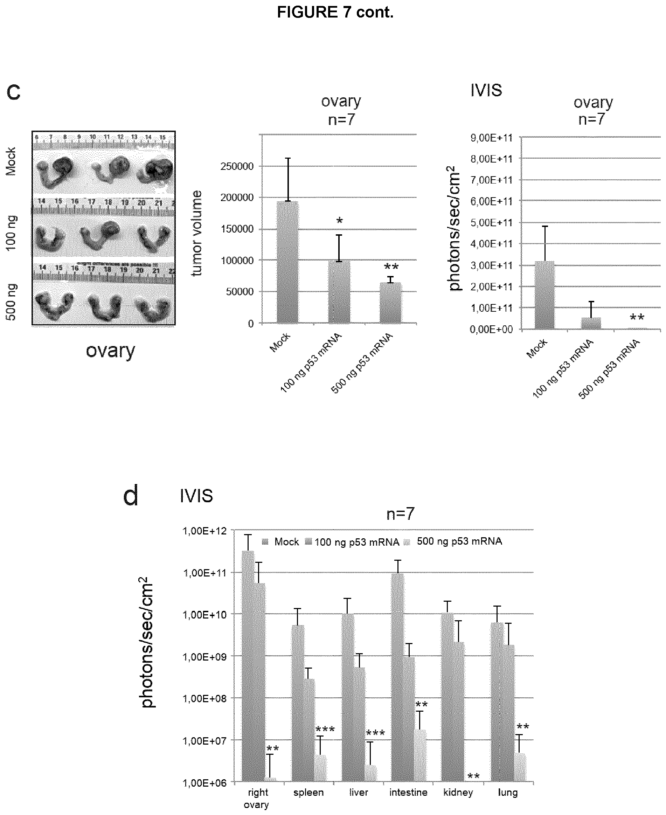

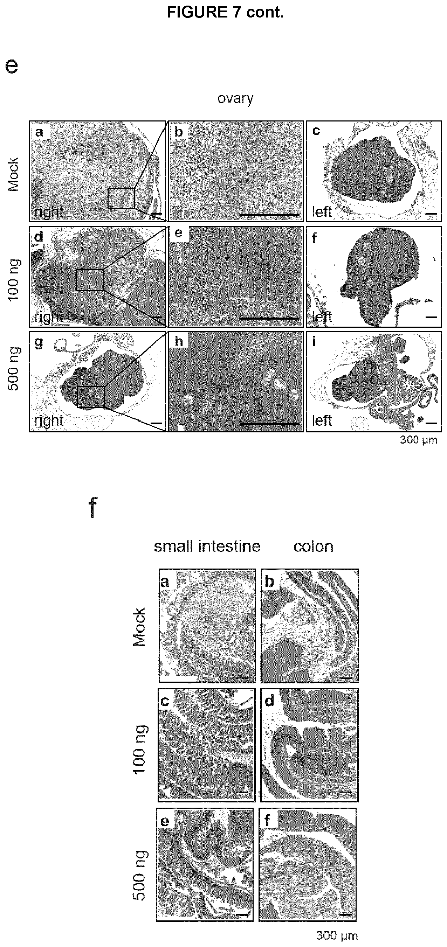

**Figure 7****:** shows application of liposomal p53 mRNA to OVCAR-8 cells prevents intrabursal growth and dissemination in an orthotopic mouse model. **a,** Schematic design of the orthotopic murine model. **b,** Bioluminescence imaging. (left) On day 0 Luc-expressing OVCAR-8 cells were transfected (mock, p53 mRNA: 100 ng, 500 ng). 1×10⁶ cells/mouse were injected into the bursa (right ovary) of nude mice on day 1 and imaged using the IVIS Lumina system following D-luciferin application. 8 weeks following orthotopic application of OVCAR-8 cells (mock- or p53 mRNA-treated) representative images of mice (one/week) at baseline are shown. Photon flux is indicated in the pseudocolored heat map. (right) BLI was performed at the indicated time points and data were presented in a graphic. Photon flux was normalized to baseline values obtained from mice on day 1.**P* < 0.05, ***P* < 0.01, Wilcoxon test, unpaired and two-tailed. **c,** (left) Representative photographs of removed ovaries 8 weeks after intrabursal application of mock- or p53 mRNA-transfected (100 ng, 500 ng) OVCAR-8 cells in mice (*n*=8 for each group). (middle) Analysis of extracted ovaries. The dose-dependent tumor volumes and (right) BLI data are represented as bar graph. **P* < 0.05, ^{**}*P*< 0.01, Wilcoxon test, unpaired and two-tailed. **d,** BLI of removed organs 8 weeks after intrabursal application of mock- (*n* = 8), or p53 mRNA-transfected (100 ng, 500 ng) OVCAR-8 cells (*n* = 8 mice for each group) represented as bar graph. ***P* < 0.01, ****P* < 0.001, Wilcoxon test, unpaired and two-tailed. **e,** Hematoxylin/Eosin-stained sections of ovaries injected with p53 mRNA-transfected (100 ng, 500 ng) (n=8) or mock- (n = 8) OVCAR-8 cells at the end of the experiment (8 weeks). Scale bars, 300 µm. **f,** Hematoxylin/Eosin-stained sections of metastases at the small intestine and colon following intrabursal injection of mock-, or p53 mRNA-transfected (100 ng, 500 ng) OVCAR-8 cells (after 8 weeks), each representative of eight. Scale bars, 300 µm.

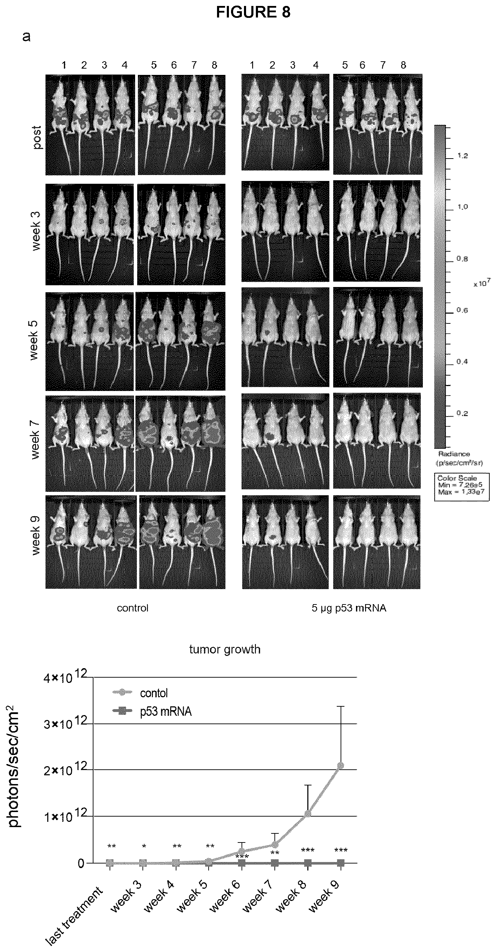

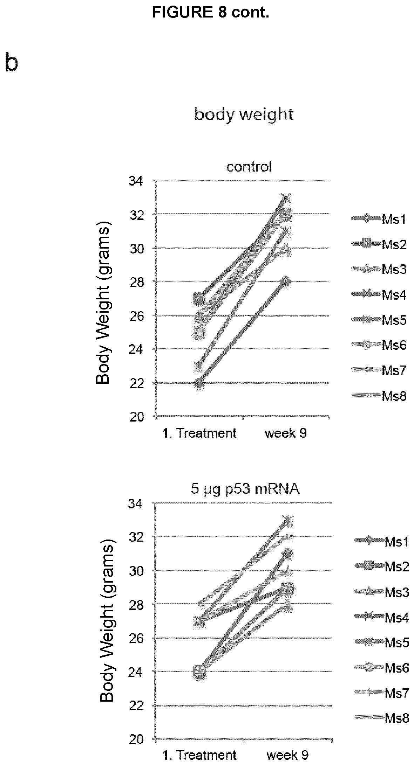

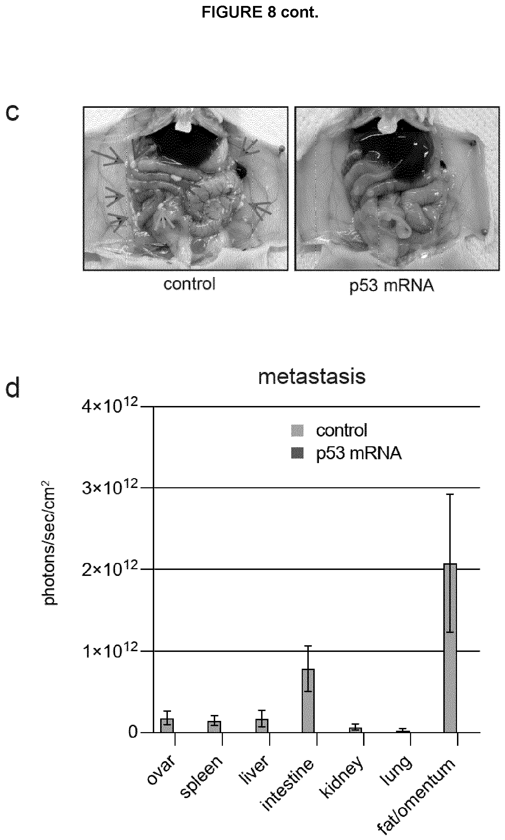

**Figure 8****:** shows i.p. injection of OVCAR-8 cells followed by i.p. treatment of mice with liposomal p53 mRNA prevents tumor formation and organ dissemination in an orthotopic mouse model. **a,** (upper) Bioluminescence imaging of luciferase-expressing metastatic OVCAR-8 cells-bearing mice upon treatment with liposomal p53 mRNA. 2×10⁶OVCAR-8 cells stably expressing luciferase were i.p. injected followed by treatment with liposomal p53 mRNA for 3 weeks (2x 5 µg liposomal p53 mRNA/week), (right) The color scale represents p/sec/cm²/steradian. (lower) Tumor burden of control and liposomal p53 mRNA-treated nude mice determined weekly by bioluminescence over a period of 9 weeks. **P* < 0.05, ***P* < 0.01, ****P* < 0.001, Wilcoxon test, unpaired and two-tailed. **b,** Determination of the body weight over the entire observation period. **c,** Inspection of peritoneal structures and organs for tumor dissemination following laparotomy. **d,** Bioluminescence imaging of removed murine organs (control vs. p53 mRNA treatment).

The sequences show:
**SEQ ID NO. 1 shows the amino acid sequence of the canonical p53 isoform in humans:**
SEQ ID NO. 2 shows the amino acid sequence of the flagged tagged p53 humans used in the examples: 
MDYKDHDGDYKDHDIDYKDDDDKMEEPQSDPSVEPPLSQETFSDLWKLLPENNVLSP
LPSQAMDDLMLSPDDIEQWFTEDPGPDEAPRMPEAAPPVAPAPAAPTPAAPAPAPSWPLSSSV
PSQKTYQGSYGFRLGFLHSGTAKSVTCTYSPALNKMFCQLAKTCPVQLWVDSTPPPGTRVRA
MAIYKQSQHMTEVVRRCPHHERCSDSDGLAPPQHLIRVEGNLRVEYLDDRNTFRHSVVVPYE
PPEVGSDCTTIHYNYMCNSSCMGGMNRRPILTIITLEDSSGNLLGRNSFEVRVCACPGRDRRT
EEENLRKKGEPHHELPPGSTKRALPNNTSSSPQPKKKPLDGEYFTLQIRGRERFEMFRELNEA
LELKDAQAGKEPGGSRAHSSHLKSKKGQSTSRHKKLMFKTEGPDSD.
SEQ ID NO: 3 shows the mRNA sequence of the p53 Expression construct of the invention.

atggactacaaagaccatgacggtgaBataaagatcatgatatcgaBacaaggatgacgatgacaagATGGAGG
AGCCGCAGTCAGATCCTAGCGTCGAGCCCCCTCTGAGTCAGGAAACATTTTCAGACCTATG
GAAACTACTTCCTGAAAACAACGTTCTGTCCCCCTTGCCGTCCCAAGCAATGGATGATTTGA
TGCTGTCCCCGGACGATATTGAACAATGGTTCACTGAAGACCCAGGTCCAGATGAAGCTCC
CAGAATGCCAGAGGCTGCTCCCCCCGTGGCCCCTGCACCAGCAGCTCCTACACCGGCGG
CCCCTGCACCAGCCCCCTCCTGGCCCCTGTCATCTTCTGTCCCTTCCCAGAAAACCTACCA
GGGCAGCTACGGTTTCCGTCTGGGCTTCTTGCATTCTGGGACAGCCAAGTCTGTGACTTG
CACGTACTCCCCTGCCCTCAACAAGATGTTTTGCCAACTGGCCAAGACCTGCCCTGTGCAG
CTGTGGGTTGATTCCACACCCCCGCCCGGCACCCGCGTCCGCGCCATGGCCATCTACAAG
CAGTCACAGCACATGACGGAGGTTGTGAGGCGCTGCCCCCACCATGAGCGCTGCTCAGAT
AGCGATGGTCTGGCCCCTCCTCAGCATCTTATCCGAGTGGAAGGAAATTTGCGTGTGGAGT
ATTTGGATGACAGAAACACTTTTCGACATAGTGTGGTGGTGCCCTATGAGCCGCCTGAGGT
TGGCTCTGACTGTACCACCATCCACTACAACTACATGTGTAACAGTTCCTGCATGGGCGGC
ATGAACCGGAGGCCCATCCTCACCATCATCACACTGGAAGACTCCAGTGGTAATCTACTGG
GACGGAACAGCTTTGAGGTGCGTGTTTGTGCCTGTCCTGGGAGAGACCGGCGCACAGAG
GAAGAGAATCTCCGCAAGAAAGGGGAGCCTCACCACGAGCTGCCCCCAGGGAGCACTAA
GCGAGCACTGCCCAACAACACCAGCTCCTCTCCCCAGCCAAAGAAGAAACCACTGGATGG
AGAATATTTCACCCTTCAGATCCGTGGGCGTGAGCGCTTCGAGATGTTCCGAGAGCTGAAT
GAGGCCTTGGAACTCAAGGATGCCCAGGCTGGGAAGGAGCCAGGGGGGAGCAGGGCTC
ACTCCAGCCACCTGAAGTCCAAAAAGGGTCAGTCTACCTCCCGCCATAAAAAACTCATGTT
CAAGACAGAAGGGCCTGACTCAGACTGA

### EXAMPLES

Certain aspects and embodiments of the invention will now be illustrated by way of example and with reference to the description, figures and tables set out herein. Such examples of the methods, uses and other aspects of the present invention are representative only, and should not be taken to limit the scope of the present invention to only such representative examples.

The examples show:

### Example 1: Mammalian vector-based expression of p53 induces apoptosis in HGSOC cells.

As no sufficiently specific drug is available yet to rescue functional wt p53 in cancer cells bearing a loss-of-function p53 mutant, we considered reactivating p53 by different experimental strategies in HGSOC cell lines and primary OC cells. First, 5 known OC cell lines were studied for p53 expression. The expression of p53 was not detected in SKOV3 and OVCAR5, in line with previous observations that classified them as p53 null. In contrast, OVCAR-3, -4, and -8 cells showed abundant p53 protein expression due to mutations at R248Q, L130V, or a single-nucleotide mutation at a splice junction (c.376-1G>A), respectively. For further analysis, we selected different HGSOC cell lines including OVCAR-8, which harbors different genomic alterations that we confirmed by sequencing. Exogenous expression of Flag-tagged wt p53 driven by a mammalian vector system for 48 h in mutant *TP53*-bearing OVCAR-8 reduced significantly cell proliferation compared to controls. Evidence for the re-activation of p53 activity is the induction of a G0/G1 cell cycle arrest. Thus, we examined cells transfected with rising amounts of the wt p53-expressing vector by flow cytometry 24 h following transfection and observed a moderate increase in the G0/G1 population accompanied by a rise in the percentage of apoptotic cells in the sub-G1 phase and by significantly increased levels of caspase 3/7 activity. The determination of early apoptotic cells that are Annexin V-positive and PI-negative (Annexin V-FITC⁺/PI⁻) and late (end-stage) apoptotic cells that are Annexin V/PI-double-positive (Annexin V-FITC⁺/PI⁺) revealed increasing levels of apoptosis in OVCAR-8 cells expressing rising levels of wt p53. Western blot (WB) experiments for the detection of cleaved Caspase-3 and PARP corroborated increasing apoptosis. To further assess the cell cycle progression in OVCAR-8 cells, we performed WB studies on critical regulators of cell cycle progression. Inhibitors of CDKs (p16, p27) and one of the most studied p53-target genes, p21, were upregulated upon a vector-prone increase of p53 expression. PLK1 and CDK1, two key players in cell cycle regulation, were downregulated. *Next, we demonstrated* the cytotoxicity of wt p53 in a dose-dependent manner through *in vitro* **assays** assessing the colony-forming ability compared to controls. Similar experiments in additional HGSOC cell lines OVCAR-3, and -5 cells corroborated growth inhibition, cell cycle arrest, induction of apoptosis, and reduction of the colony forming ability upon wt p53 expression. Collectively, these data indicated that rescuing p53 function by vector-based re-expression of wt p53 induces cell cycle misregulation and death in HGSOC cells in a dose-dependent manner.

### Example 2: Design, generation, and characterization of IVT mRNAs in HGSOC cell lines.

Recently, the therapeutic use of mRNA as a vaccine has fueled great hope to fight a spectrum of infectious diseases. However, whether this class of biological agents might represent a new generation of "magic bullets" for precision medicine targeting cancer as postulated more than 100 years ago by Paul Ehrlich remains elusive. To address this unanswered question, we used an IVT system based on a bacterial vector including a T7 RNA Polymerase promotor for the transcription of mRNAs (human p53, firefly Luciferase (Luc), Enhanced Green Fluorescent Protein (EGFP)) considering aspects of translational efficacy and decay rates of mRNA. After IVT, the thorough purification of mRNAs included the use of DNase for the degradation of the DNA template and of a spin column (silica membrane) to remove the digested template, excess NTP's, salts, and unused capping analog. IVT-mRNAs like IVT-Flag-tagged p53 mRNA, dubbed from here on as p53 mRNA, were analyzed by agarose gel electrophoresis for quality control.

Based on the clinical advantages of liposomes as carriers of chemotherapeutics over conventional chemotherapy due to reduced therapeutical side effects and increased anti-cancer activity, we exploited a liposomal system to test its supportive quality for the delivery of IVT-mRNA (EGFP-mRNA, luc-mRNA) to HGSOC cells. To this end, we first examined the transfection efficiency in different HGSOC cell lines using control EGFP-mRNA encapsulated in positively charged lipid molecules (liposomes). To study the uptake on a quantitative basis, EGFP expression was measured by flow cytometry as a surrogate marker for transfection efficiency. At 24 h following transfection of EGFP-mRNA (0.1-2 µg), a dose-dependent increase of EGFP expression was detected. While 0.25 µg EGFP-mRNA was sufficient for an expression in 90% of cells, at doses ≥0.5 µg 97-100% of cells showed EGFP fluorescence supporting an excellent transfection efficiency. This observation could be confirmed by WB showing high levels of EGFP at doses of ≥0.25 µg EGFP-mRNA. In time kinetics using 1 µg EGFP-mRNA, a high percentage of EGFP-expressing cells (92.2-99.7%) persisted for 10 days (d) following transfection and declined to baseline levels (≤5.5%) between d 15 and 20. EGFP expression could be corroborated for a period of 7-8 d by WB. As a second control IVT-mRNA, Luc-mRNA transfection was monitored by WB and by activity measurements showing high enzymatic activity in cells transfected with 0.25-2 µg Luc mRNA.

To further validate the usefulness of our approach for the expression of a protein of choice driven by IVT-mRNA in OC, we tested additional cells lines (OVCAR-3, -4, -5, -8) by immunofluorescence, by flow cytometry and WB. Strong expression (≥70%) could be detected in OVCAR-5 cells and very strong expression (≥95%) in OVCAR-3, -4 and -8 cells transfected with 1 µg EGFP-mRNA. Collectively, different experiments demonstrated the high efficiency of liposomal-based IVT-mRNA transfection and expression irrespective of the OC cell line or type of mRNA.

### Example 3: Characterization of mRNA expression in primary normal ovarian and primary HGSOC cells.

Next, we investigated the time kinetics of EGFP- and Luc-mRNA expression in primary HGSOC cells. EGFP expression using 1 µg mRNA could be detected in FACS studies on day 1 with 85% positive cells descending to 74% on day 11. The verification by WB showed EGFP expression for at least 7 d. Upon transfection of Luc-mRNA the corresponding enzymatic activity was detectable for 7 d, and WB signals for 4 d. Next, *tumor organoids* which are promising models in cancer research were prepared from primary HGSOC tissues, metastases, and normal ovarian tissues. Organoids from primary tumors and metastatic sites showed efficient Luc activity or EGFP expression for up to 5 d. Luc activity in normal ovarian cells was detectable only for 1-2 d. To monitor the duration of Luc activity in mice by *in vivo* Bioluminescence Imaging (BLI), we transfected OVCAR-8 cells with Luc-mRNA for subsequent application to mice: (i) orthotopic implantation of 1×10⁶ OVCAR-8 cells transfected with 2 µg Luc-mRNA, (ii) i.p. injection of 2x10⁶ OVCAR-8 cells transfected with 4 µg Luc-mRNA or (iii) i.p. injection of 10 µg liposomal Luc-mRNA. Luciferase activity was detectable for 3 d by BLI in all 3 experimental settings. Taken together, liposomes were well suited for an efficient and prolonged IVT-mRNA expression in primary human cells (2D-, 3D) and in mice.

### Example 4: mRNA-based rescue of p53 function in HGSOC cell lines induces cell cycle arrest and cell death.

Although *TP53* mutations are so prevalent in HGSOC, there is of yet no clinical drug approved for rescuing the function of wt p53. Thus, we aimed at rescuing p53 function in different preclinical models by transfecting HGSOC cell lines and primary cells (2D cultures, organoids) with IVT-mRNA coding for p53. Low amounts of p53 mRNA (10-250 ng) induced strong p53 protein expression in OVCAR-8 cells (Fig. 1a). A prominent increase of 22.45% of cells in G0/G1 accompanied by a decrease of 57.14% in S phase was observed at 0.1 µg p53 mRNA 24 h post-transfection compared to controls (Fig. 1b) as an indication for re-activation of p53 activity corroborating our results using a vector-based re-expression of p53. Based on the high levels of p53 expression using p53 mRNA compared to the vector-based experiments, a strong induction of the cell cycle inhibitors p21, p16, and p27 was readily visible at low doses of p53 mRNA (≥0.1 µg) in parallel to the downregulation of critical cell cycle regulators like PLK1, Aurora A, CDK1, and Cyclin A/B (Fig. 1c, upper). These observations correlated with significant drop viability in cellular viability by ∼2-fold after 48 h at a dose of 0.5 µg (Fig. 1d) accompanied by different criteria of cell death: rising levels of Puma, Noxa, and Fas (Fig. 1c, lower), Caspase-3 and PARP (Fig. 1c, lower), Caspase 3/7 activities (Fig. 1e) and Annexin staining (Fig. 1f). Synchronized, p53 mRNA-transfected cells confirmed clearly the dose-dependent increase in G1 (Fig. 1g). A loss of colony formation ability of OVCAR-8 cells increased with rising doses of p53 mRNA (Fig. 1h). Moreover, we tested p53-null HGSOC cells such as OVCAR-5 for their sensitivity to the re-expression of wt p53. Within 24 h following p53 mRNA transfection prominent signs of cell death including strong PARP cleavage, high levels of cells in sub-GO, and high levels of Caspase 3/7 activity could be observed. Taken together, our observations indicated that rescuing p53 function by transfection of wt p53 mRNA induces cell death in different HGSOC cell lines.

### Example 5: Reinstating p53 reduces numerical chromosomal instability (nCIN) in the HGSOC cell line OVCAR-8.

Chromosomal instability (CIN), or karyotype instability, is a fundamental feature of OC, with most human OC cells exhibiting aneuploidy. The OVCAR-8 cell line is not an exception to this, as it has been classified as a hyperdiploid cell line with high numerical complexity and a modal number of chromosomes ranging from 50 to 59. Our study aimed to determine whether restoring functional wt p53 could counteract nCIN and reduce the number of chromosomes in cells to a near diploid status. To this end, OVCAR-8 cells were treated every two days for 28 days with low doses of p53 mRNA, and at the end of the incubation period, the distribution of chromosomes was assessed using chromosome spreadings (Fig. 2, d). The expression of p53 significantly decreased the total chromosome number in cells treated with p53 mRNA compared to controls (Fig. 2c, upper). The mean chromosome number was reduced from 53 to 47 upon re-expression of wt p53, providing additional evidence of the critical role played by p53 in maintaining genomic integrity in HGSOC cells (Fig. 2c, lower).

### Example 6: mRNA-based rescue of p53 function in primary HGSOC cells induces cell cycle arrest and cell death.

To further validate the rescue of p53 in a preclinical scenario, we compared the effects of p53 mRNA on primary human cells (normal, cancer) (Fig. 3a). p53 expression driven by transfected p53 mRNA could be detected in different primary samples, except for keratinocytes (Fig. 3a). A first comparison of HGSOC and the corresponding normal ovarian tissue from the same patient revealed a higher expression of p53 in tumor cells despite equal amounts of transfected IVT-mRNA (Fig. 3a). The analysis of additional tissues demonstrated that the expression of p53 (Fig. 3b upper, lower left) and the activity of Caspase 3/7 in all tumor samples (Fig. 3b, lower right) were significantly above the corresponding levels in normal ovarian cells. In contrast to this, a similar experiment with IVT-GFP-mRNA resulted in nearly the same levels of GFP expression upon transfection of equal amounts of IVT-mRNA. Testing the transfection efficacy of fluorescent mRNA in normal and HGSOC cells a difference could not be detected suggesting that diverging transfection efficiencies did not seem to be the reason for different p53 levels (tumor vs. normal). The WB examination revealed the activation of the cell cycle inhibitors p21, p16, and p27 (Fig. 3c) and apoptosis (Fig. 3d), to be stronger in p53 mRNA-transfected primary HGSOC cells compared to normal counterparts, correlating with a more pronounced reduction in the number of HGSOC colonies (Fig. 3e). The analysis of HGSOC organoids from patients P1-3 demonstrated also a significant increase of Caspase 3/7 activity upon p53 mRNA-treatment under 3D cell culture conditions (Fig. 3f). In summary, although the expression of p53 reduced the viability of both types of primary cells (normal vs. HGSOC), the effect was significantly more pronounced in primary HGSOC cells compared to normal counterparts (normal ≈21-fold vs. malignant ≈150-fold) which could be due to elevated levels of p53 in tumor cells.

### Example 7: Transcriptome alterations in OVCAR-8 cells upon the rescue of wild-type p53 functions.

Our observations of reduced proliferative activity and increased cell death in cell lines and primary cells of HGSOC origin supported the model of rescued p53 functions upon re-expression of p53 by mRNA transfection. To deepen our understanding of the underlying biological processes and to address this aspect in a relatively unbiased manner, we transfected OVCAR-8 cells with 1 µg p53 mRNA or with mock control (EGFP mRNA) in three independent experiments. A total of 25,540 human genes were profiled by mRNA sequencing 24 h after transfection. Both groups were compared by unpaired *t*-tests considering the group variances. All calculated p values were corrected using the Benjamini and Hochberg false discovery rate (FDR) method to discard false positives by the fact of performing multiple tests. Differentially expressed genes (DEG) between both groups were selected according to the criteria of FOR *q* <0.05 and absolute fold change >1.5. The transcriptomics analysis revealed 3,061 transcripts that were upregulated, and 1,662 transcripts that were downregulated (Fig. 4a, b). These significant genes were further analyzed with Ingenuity Pathway Analysis (IPA) software to identify overrepresented canonical pathways associated with p53 mRNA transfection in OVCAR-8 cells (Fig. 4c).

Consistent with the pro-apoptotic functions of p53 as a tumor suppressor involved in the induction of cell cycle arrest and apoptosis, our Ingenuity pathway analysis revealed an overrepresentation of several related pathways upon p53 mRNA transfection, such as 'p53 Signaling', 'Apoptosis Signaling' and several 'cell cycle' pathways (Fig. 4c). To confirm these findings, we investigated all genes involved in the IPA 'p53 Signaling' pathway and we realized that most of them (51/81, 63%) were differentially expressed with FOR *q* <0.05 and absolute fold change >1.5 (Fig. 4d). Notably, most of the known downstream targets of p53 signaling (96%) were coordinately upregulated by the transfection of p53 mRNA. We further validated these results by performing WB on selected proteins from the 'p53 Signaling pathway', including p21 (*CDKN1A*)*,* MDM2, Puma (*BBC3*), p73, FAS (CD95) and CDK1 (Fig. 4d). Their expression in OVCAR-8 cells was substantially up- or downregulated by p53 mRNA in agreement with the transcriptomic analysis. Regarding the 'Apoptosis Signaling' pathway, most genes of this pathway (42/65, 65%) were significantly deregulated by p53 mRNA transfection. We extensively validated 13 of these proteins by WB to demonstrate that p53 mRNA transfection induces apoptosis signaling in OVCAR-8 cells.

Several 'cell cycle' pathways, including 'Cell Cycle Control of Chromosomal Replication', 'Cell Cycle: G2/M DNA Damage Checkpoint Regulation' and `Cyclins and Cell Cycle Regulation' were significantly overrepresented, as well as 'Mitotic Roles of Polo-Like Kinase', and showed a global downregulation (negative z score) (Fig. 4c). Most of the genes from these pathways were differentially expressed and were validated by WB, confirming the downregulation of cell cycle and mitotic roles of PLK1 by p53 mRNA transfection. Remarkably, the cellular senescence pathway was also upregulated by p53 mRNA transfection and might contribute to the observed cell death of OVCAR-8 cells along with the induction of apoptosis.

Next, we carried out a transcriptome analysis between p53 mRNA and mock-transfected primary HGSOC cells from patient 1 (Fig. 5a). The high variability among samples resulted in low significance after FOR correction for multiple testing. Thus, we considered the significance for transcripts of primary HGSOC cells with *P* <0.01 and FC >1.5. A total of 156 transcripts were upregulated and 115 were downregulated (Fig. 5b, c). Several genes from primary HGSOC cells were significant and in common with the pathways identified in OVCAR-8 cells (Fig. 5d), supporting the relevance of both data sets.

Consistent with the paramount function of p53 for the development of OC, our Ingenuity pathway analysis showed multiple transcriptional alterations with the "ovarian cancer signaling" pathway. Many genes of this pathway (55/76, 72 %) were significantly deregulated (Fig. 6a, b). As an example, we found that many genes of Wnt/β-catenin signaling were deregulated. This pathway promotes chemoresistance, metastasis, and cancer stem cell self-renewal of OC by aberrant activation of Wnt/β-catenin signaling leading to the hyperactivity of β-catenin. p53 mRNA treatment resulted in an upregulation of AXIN1, which functions as a scaffolding protein for components of the β-catenin destruction complex including APC, CK1PP2A, and GSK3β. In addition, deregulation was observed for members of the frizzled receptor-, Wnt-, and TCF/LEF-families (Fig. 6a, b). Altogether, these transcriptional alterations led to a moderate reduction of β-catenin in p53 mRNA-treated cells (Fig. 6b) and to descending activity upon transfection with rising amounts of p53 mRNA in measurements of Wnt signaling using a TCF/LEF reporter kit.

### Example 8: Orthotopical application of p53 mRNA-treated OVCAR-8 cells results in a dose-dependent reduction of tumor growth in an HGSOC Xenograft model.

To explore the viability of p53 mRNA-treated OC cells in a physiological microenvironment, an orthotopic disease model was studied. First, we tested whether stably Luciferase (Luc)-expressing OVCAR-8 cells (OVCAR-8/Luc) show an altered response to p53-reactivation compared to wt cells. While the treatment with 100 ng p53 mRNA correlated only to moderate cell death as evidenced by low expression of apoptotic markers/activation, low Caspase 3/7 activity, and moderate inhibition of cell growth as demonstrated by the colony formation, the transfection with 500 ng p53 mRNA induced a robust apoptotic response and a strong drop in the colony forming efficacy. Thus, the stable expression of Luc in OVCAR-8 cells did not alter the response to the reactivation of p53 by p53 mRNA compared to wt OVCAR-8 cells. One day prior to the Xenograft experiment, OVCAR-8/Luc cells were mock- or p53 mRNA (100 ng, 500 ng)-transfected (Fig. 7a). To establish the orthotopic model, a Matrigel/OVCAR-8 cell suspension was injected into the right ovary of each mouse. The determination of tumor volumes 1x/week for an observation period of 10 weeks by BLI demonstrated growth retardation in tumors originating from cells treated with 100 ng p53 mRNA and a complete block of cells treated with 500 ng p53 mRNA (Fig. 7b). To verify measurements of Luc-activity *in vivo,* mice were sacrificed at the end of the study and subjected to an anatomical/histological examination of all organs by two independent veterinary pathologists. The inspection of removed ovaries based on the determination of volume (Fig. 7c, left, middle), and BLI measurements (Fig. 7c, right) confirmed the dose-dependent reduction of tumor size by p53 mRNA compared to *in vivo* BLI measurements (Fig. 7b). The histological examination revealed huge tumor masses and profoundly altered morphology due to vast carcinomal tissue in the right ovaries injected with mock-treated OVCAR-8/Luc cells (Fig. 7e: panels a, b). Within the 100 ng p53 mRNA-treated group, right ovaries exhibited tumor tissue (Fig. 7e: panels d, e) or normal histology. Right ovaries within the 500 ng p53 mRNA-treated group and all other organs showed normal histology (Fig. 7e: panels g, h). In all treatment groups (mock, 100 ng, 500 ng p53 mRNA), left ovaries which did not receive OVCAR-8/Luc cells showed normal histology with follicular and luteal structures as well as stromal tissue (Fig. 7e: panels c, f, i). While in mice injected with mock-treated cells, metastases could be detected at the intestine, the metastatic activity in the 100 ng-group was low and absent in the 500 ng-group. Taken together, the animal data revealed the dose-dependent growth retardation of p53 mRNA-treated OVCAR-8/Luc cells under orthotopic conditions.

### Example 9: i.p. application of OVCAR-8 cells followed by i.p. treatment with p53 mRNA inhibits dissemination of tumor cells.

In advanced stages of OC, an intra-abdominal spread of tumor cells is common. Therefore, the goal of the debulking surgery is the complete resection of all visible tumors³⁷. Still, a high percentage of all OC patients will succumb to their disease, indicating that the remaining cancer cells have a fatal impact on the clinical outcome. To further validate the clinical relevance of p53 mRNA for HGSOC, we tested whether scattered tumor cells within the peritoneum are accessible for liposomal p53 mRNA. The i.p. administration of drugs for OC was shown to be more efficacious compared to the systemic administration and was thus recommended by the National Cancer Institute. Thus, we injected 2x10⁶ OVCAR-8/Luc cells i.p. and administered p53 mRNA via the intraperitoneal route. Mice were treated for 3 weeks with 2 i.p. injections/week with p53 mRNA (33 mg/kg) or control. While OVCAR-8/Luc cells treated with the control grew exponentially, the i.p. treatment with liposomal p53 mRNA blocked the tumor cell growth completely (Fig. 8a). During the entire observation period, the body weight in both groups developed normally (Fig. 8b).

In control animals receiving i.p. OVCAR-8/Luc cells and i.p. mock treatment the gross anatomical analysis revealed tumor masses on peritoneal surfaces (Fig. 8c, d). Most of these metastases were only weakly attached to the organs. Big tumor masses were also detected in the fatty tissues such as the omentum, the ovarian fat pad, and the mesentery (Fig. 8c, d). In control animals, enlargement of organs, e.g. spleen, kidney, and uterus was observed. Especially larger tumor masses attached to the small and large intestines led to adhesion between intestinal structures implicating strictures of the gut. IVIS measurements of the omentum and intestine revealed the most prominent signals (Fig. 8d). In mice treated with p53 mRNA, the gross anatomical analysis did not reveal tumor masses in the peritoneal cavity, and all organs appeared normal by visual inspection. IVIS measurements of those organs showed no signal (Fig. 8d).

## Claims

1. **A ribonucleic acid (RNA) compound for use in the treatment of High-grade serous ovarian cancer (HGSOC)** in a subject, wherein the RNA compound encodes an amino acid sequence of a human p53 protein.

2. The RNA compound for use of claim 1, wherein the RNA compound encoding the amino acid sequence comprises the nucleotide sequence of SEQ ID NO: 1 (RNA sequence preferred), or a nucleotide sequence having at least 80%, 85%, 90%, 95%, 96%, 97%, 98%, or 99% identity to the nucleotide sequence of SEQ ID NO: 1.

3. The RNA compound for use of claim 1 or 2, wherein the RNA compound is an in vitro transcribed (IVT) RNA or is a synthetically polymerized RNA.

4. The RNA compound for use of any one of claims 1 to 3, wherein at least one nucleotide is a modified nucleotide, such as pseudo uridine or 5-methylcytidine, for example wherein the modified nucleotide is a replacement of at least one uracil with pseudo uridine, preferably is a replacement of all uracil positions with pseudo uridine.

5. The RNA compound for use of any one of claims 1 to 4, wherein the RNA compound is administered to the subject by administering a pharmaceutically acceptable liposomal formulation comprising the RNA-compound.

6. The RNA compound for use of any one of claims 1 to 5, wherein the cancer is metastatic.

7. The RNA compound for use of any one of claims 1 to 6, wherein the cancer is relapsed.

8. The RNA compound for use of any one of claims 1 to 7, wherein the subject received a surgery.

9. The RNA compound for use of any one of claims 1 to 8, wherein the subject received tumor debulking therapy in the intra peritoneal cavity.

10. The RNA compound for use of any one of claims 1 to 9, wherein the RNA compound is administered to the subject intraperitoneal.

11. The RNA compound for use of any one of claims 1 to 10, wherein the subject received or receives at least one additional anticancer therapy.

12. The RNA compound for use of any one of claims 1 to 11, wherein the proliferative disease is HGSOC, the subject received debulking therapy, and wherein the RNA compound is administered into the intraperitoneal cavity.

13. **A pharmaceutical composition,** the composition comprising the RNA compound recited in any one of claims 1 to 12, and at least one pharmaceutically acceptable excipient.

14. The pharmaceutical composition of claim 13, which is a liposomal formulation of the RNA compound.

15. The pharmaceutical composition of claim 13 or 14, for a use in therapy as recited in any one of claims 1 to 11.
